Europäisches Patentamt
European Patent Office
Office européen des brevets

(11) Publication number: **0 002 681**
**A2**

# EUROPEAN PATENT APPLICATION

(21) Application number: 78101549.0

(22) Date of filing: 04.12.78

(51) Int. Cl.²: C 07 C 51/20, C 07 C 53/08, B 01 J 23/22

(30) Priority: 12.12.77 US 859895

(43) Date of publication of application: 11.07.79 Bulletin 79/14

(84) Designated Contracting States: BE DE FR GB NL

(71) Applicant: CELANESE CORPORATION, 1211 Avenue Of The Americas, New York New York 10036 (US)

(72) Inventor: Slinkard, William E., 4521 Sheffield, Corpus Christi Texas 78411 (US)
Inventor: Baylis, Anthony B., 73 Robbins Avenue, Berkeley Heights New Jersey, 07922 (US)
Inventor: Aguilo, Adolfo, 4606 Oxford, Corpus Christi Texas 78411 (US)
Inventor: DeGroot, Peter B., 906 Catalina, Corpus Christi Texas 78411 (US)
Inventor: Hughes, Michael P., 2800 Teakwood, Odessa Texas 78411 (US)

(74) Representative: Keller, Johanna Carola et al, Deichmannhaus am Hauptbahnhof, D-5000 Köln 1 (DE)

(54) **Vanadium oxide catalyst, its preparation and its use in the high pressure vapor phase oxidation of lower aliphatic hydrocarbons.**

(57) The present invention provides a superior vanadium oxide catalyst, its preparation and its use in the high pressure vapor phase oxidation of lower aliphatic hydrocarbons such as n-butane to corresponding monocarboxylic acids such as acetic acid. The catalyst is prepared by thermally decomposing in an inert atmosphere salts of vanadium and multibasic organic acids. The use of said catalyst takes place in the hydrocarbon rich region in the presence of steam.

EP 0 002 681 A2

# VANADIUM OXIDE CATALYST, ITS PREPARATION AND ITS USE IN THE HIGH PRESSURE VAPOR PHASE OXIDATION OF LOWER ALIPHATIC HYDROCARBONS

## BACKGROUND OF THE INVENTION

Processes for producing lower aliphatic monocarboxylic acids such as acetic acid by the vapor phase oxidation of lower aliphatic hydrocarbons are known. For example, acetic acid is prepared by the vapor phase oxidation of butane according to the following equation:

$$C_4H_{10} + 5/2\ O_2 \rightarrow 2CH_3COOH + H_2O$$

However, processes for the oxidation of hydrocarbons in the vapor phase by means of oxygen-containing gases have not proven entirely satisfactory primarily due to the excessive formation of undesirable carbon oxides, and to the difficulty in maintaining control of the highly exothermic oxidation reaction. U. S. Patent 3,395,159 provides an improved process wherein the oxidation of hydrocarbons is performed in a reactor system having fused vanadium oxide catalyst coated on the inner surface of the reactor, which system has the

advantage of better temperature control and isothermal operation. The use of early catalysts, such as vanadium pentoxide either supported or unsupported, for the vapor phase oxidation of lower aliphatic hydrocarbons generally results in yields and process efficiencies which fall substantially short of economic potential. Also, the resulting products are often impure due to a lack of selectivity when such catalysts are employed.

There are three criteria which are used to judge the performance of a catalyst to be used in the oxidation conversion of lower aliphatic hydrocarbons, for example, butane to corresponding monocarboxylic acids such as acetic acid. For instance, in the oxidation of normal butane, it is highly desirable to have a low overall efficiency to the unsaturated equivalent, i.e., butene and low efficiency to undesirable by-products, carbon oxides. Additionally, one desires to have a highly efficient conversion of any butene formed to acetic acid and finally a very rapid reaction rate of the butene to acetic acid to prevent buildup of butene within the reactor.

## OBJECTS AND SUMMARY OF THE INVENTION

Accordingly, a general object of the present invention is to find a catalyst which meets the above three criteria in a satisfactory manner. Another object is a process for preparing this catalyst. A more specific object is the use of the catalyst in a process for the production of acetic acid by the vapor phase oxidation of lower aliphatic hydrocarbons in a high pressure environment, and in the hydrocarbon rich region.

These objects are achieved by a process for preparing acetic acid by the vapor phase oxidation of the lower aliphatic hydrocarbon such as butane, which

process comprises reacting the lower aliphatic hydrocarbon and an oxygen-containing gas in the vapor phase, in the presence of steam and above atmospheric pressures with a catalytic amount of a decomposed salt of vanadium and multibasic organic acid preferably vanadyl oxalate.

One essence of this invention lies in the discovery that reduced vanadium oxide catalysts prepared from a precursor comprising salts of vanadium and multibasic organic acids when utilized in the vapor phase oxidation of a lower aliphatic hydrocarbon such as butane, with steam and high pressure conditions in the hydrocarbon rich region realizes high efficiency to acetic acid; low overall efficiency to butenes and rapid conversion of recycled butenes to acetic acid which avoids butene buildup within the reactor.

It is not new that vanadium salts derived from the reaction of vanadium with multicarboxylic acids can be decomposed to form reduced vanadium oxides, but the marked superiority of vanadium oxides formed in this manner as catalysts for hydrocarbon oxidation to produce, for example, acetic acid has not been known heretofore. For example, butene formation, which is an index of catalyst quality in this reaction, is markedly less with the present catalysts as compared with others which are formed even by such closely-related methods as decomposing vanadyl formate. The reason for these differences is not known.

Furthermore, it has been discovered that the ability to obtain decreased selectivities to carbon oxides by lowering temperature in oxidizing $C_4$ hydrocarbons over reduced vanadium catalysts is limited due to a competing reaction of catalyst oxidation at low temperatures when operating at above-atmospheric pressures, i.e., "safe" operational

minimum temperature limits exist (no catalyst oxidation) with high pressure conditions.

Other objects and advantages of the present invention will become apparent from the following description of the preferred embodiments.

## DESCRIPTION OF THE PREFERRED EMBODIMENTS

The preparation of the catalyst of the instant invention begins by preparing a water soluble salt of vanadium with a multibasic organic acid such as oxalic, tartaric, etc. Techniques for accomplishing this are well known in the art. For example, solutions of oxalic acid with water can be added to suspensions of $V_2O_5$ in water with vigorous stirring. Evaporators can be utilized to recover the crystalline salts and salts decomposed under inert atmosphere with high temperatures.

In the process using the catalyst of the present invention, a lower aliphatic hydrocarbon is reacted with an oxygen-containing gas in the presence of a catalytically effective amount of the above-described reduced vanadium oxide supported or unsupported catalyst to produce acetic acid.

By "lower aliphatic hydrocarbon" is meant any saturated or unsaturated aliphatic hydrocarbon containing from 2 to 10 carbon atoms. These lower aliphatic hydrocarbons include alkanes, alkenes and alkynes such as ethylene, propylene, butene, propane, butane, pentene, octane and their isomers. Particularly preferred aliphatic hydrocarbons are the alkanes and alkenes including propane, butane, butene, isobutane, isobutene and mixtures thereof.

The production of acetic acid from butane can give particularly advantageous results.

By the term "reduced vanadium oxide or reduced vanadium pentoxide" is meant a vanadium oxide in which the average vanadium ion valency is less than 5. A lower oxidation state of vanadium ions is an essential feature of the present invention catalyst. This is based on the observation that vanadium pentoxide (i.e., the catalyst containing vanadium ions with a valence of 5) is not an active catalyst under the process conditions to be described, and it is, therefore, advantageous to exclude vanadium pentoxide from the catalyst compositions to the greatest extent possible.

The reduced vanadium oxides employed in the catalyst of the present invention are all intermediate in average oxidation states, i.e., between $V_2O_5$ and $V_2O_3$. Although the initial charge can contain $V_2O_5$ and $V_2O_3$, X-ray diffraction studies confirm that vanadium oxide catalysts operable in the process are effectively expressed empirically as $V_3O_5$, $V_4O_7$, $V_5O_9$, $V_6O_{11}$, $V_7O_{13}$, $V_2O_4$ and/or $V_6O_{13}$. The average valence of the vanadium ions in these oxides generally ranges from 3 to about 4.5.

The oxygen necessary as a reactant in the present process may be from practically any molecular oxygen-containing gas such as molecular oxygen or air. Also, the molecular oxygen-containing gas may be one wherein molecular oxygen is mixed in varying amounts with an inert diluent gas such as nitrogen, argon, or a carbon oxide. The lower aliphatic hydrocarbon and oxygen-containing gas can be reacted within a wide range of molar ratios. However, it is an essential feature of the invention process that the quantity of oxygen gas in the feed stream be the least required to convert efficiently the hydrocarbon stream to acetic acid consistent with necessary temperature control and retention of catalyst activity. It is important that the vanadium oxide

catalyst is not oxidized to vanadium pentoxide. Even the presence of a small amount of vanadium pentoxide is effective in reducing the yield of acetic acid. The quantity of oxygen gas in the feed stream usually is maintained in the range from about 0.05 and 1 mol/mole of lower aliphatic hydrocarbon. At elevated pressures, the preferred range is from about 0.05 to about 0.30.

The present process should be carried out above atmospheric pressures, generally from greater than 1 to about 50, typically from greater than 1 to about 30 bars, and preferably from about 5 to about 20 bars.

It is, of course, highly desirable to operate the instant process under high pressure conditions for then simple cooling can render the recycle stream of butane and butene and the take-off stream of acetic acid liquid. Furthermore, high pressure operation realizes higher butane oxidation rates.

With experiments conducted at atmospheric pressure, no catalyst oxidation was apparent even when the reactor temperatures were as low as 220°C. However, when high pressure operation was commenced, as mentioned before, the ability to obtain decreased selectivities to carbon oxides by decreasing temperature in oxidizing $C_4$ hydrocarbons over reduced vanadium catalysts was limited due to a competing reaction of catalyst oxidation and therefore deactivation, at low temperatures.

In the pressure range of from about 5 to about 10 bars, significant catalyst oxidation may occur from about 220 to about 240°C. Deactivation is detectable in the 240 to 250°C. transition range. However, in reactor runs about 250°C., the catalysts of the instant invention appear to be stable to oxidation under given reaction conditions.

Visual examination of used catalyst pellets following a series of experiments at both high and low temperature conditions, suggests that the catalyst activation-deactivation process is reversible under reaction conditions with possible minor hysteresis losses.

In a preferred embodiment of the process, water is included in the feedstream in a quantity between about 0.1 and 2.0 mole per mole of lower aliphatic hydrocarbon preferably 0.5 to 1.2. The presence of water vapor in the oxidation reaction system can increase the yield of acetic acid by as much as 10% in the case where the hydrocarbon feed stream is normal butane.

Importantly, steam - which heretofore has been used in reactions such as oxidation reactions, for temperature control, i.e., to function as a diluent and control hot spots within the reactor; not only does that in the instant oxidation reaction but also, since the proposed improved catalysts for this oxidation of lower aliphatic hydrocarbons are generally in a reduced state, the steam serendipitously 1)helps maintain the vanadium oxides in a reduced and active state and 2) additionally increases selectivity of the catalyst to the desired end product, i.e., acetic acid. This, of course, was not realized in the prior art because either 1) the catalyst systems utilized in the vapor phase oxidation of lower aliphatic hydrocarbons were in a fully oxidized state or 2) the reactions were conducted at atmospheric (or below) pressures wherein the catalyst oxidation does not take place. In low pressure operations, it is believed that the reduction of the catalyst by the butane and the oxidation of the butane by air (or oxygen) vis-a-vis the oxidation of the catalyst by air (or oxygen) is such that catalyst deactivation is not a noticeable problem with low

temperature operations.

The present process is carried out at a temperature generally between 220 and about 400°C., typically from greater than 240 to about 350°C., and preferably from greater than 250 to about 300°C.

The contact time of the reactants with the catalyst is generally between about 0.1 and 100 seconds, typically between about 0.25 and 50 seconds. By contact time as used herein is meant the contact time adjusted to 25°C. and 1 atmospheric pressure (i.e., standard temperature and pressure, denoted STP). Thus, the contact time is calculated by dividing the volume of the catalyst bed (including voids) by the volume per unit time flow rate of the reactants at STP.

By using the catalyst of the present invention the process may be carried out continuously and the catalyst may be present in various forms such as in one or more fixed beds or as a fluidized system. Portions of the reactants which do not undergo reaction may be recycled if necessary. Selected intermediate products, such as butenes and acetaldehyde, are preferably recycled also. The desired acetic acid product may be separated from any impurities by condensation followed by fractionation and aqueous or non-aqueous extraction of the product from the unreacted lower aliphatic hydrocarbon. In this specification, the terms "conversion" and "efficiency" are defined as follows:

$$\text{conversion, \%} = \frac{\text{moles lower aliphatic hydrocarbon or oxygen converted}}{\text{moles lower aliphatic hydrocarbon or oxygen fed}} \times 100$$

$$\text{carbon efficiency to component i (\%)} = \frac{\text{moles component i formed} \times \text{carbon atoms per mole of component i}}{\text{total moles of carbon in all products analyzed}} \times 100$$

0002681

Acetic acid is generally produced by the present process with a conversion (based on oxygen) generally of at least 90 percent, often at least about 95 percent, a conversion based on lower aliphatic hydrocarbon (which, as noted above, is present in substantial excess) generally of at least about 1 percent, typically from about 3 to about 5 percent, and a carbon efficiency of generally at least about 50 percent, typically at least about 55 percent, often at least about 60 percent with recycled intermediates.

The recovery of the product stream and the separation of the acetic acid from the acetaldehyde, maleic acid and other by-products can be accomplished by conventional procedures. U. S. Patent 3,624,148 describes a method for the separation of acetic acid from maleic acid.

A number of multibasic organic acids may be used to prepare the desired water soluble vanadium salt such as oxalic acid, tartaric acid, succinic acid, citric acid, malonic acid, glutaric acid, adipic acid, lactic acid, and the like. The preferred acid is oxalic acid.

The present invention is further illustrated by the following examples. All parts and percentages in the examples as well as in the specification and claims are by weight unless otherwise specified. The reactants and other specific ingredients are presented as being typical, and various modifications can be devised in view of the foregoing disclosure within the scope of the invention.

## CATALYST PREPARATION

The following example illustrates the preferred method for making the catalyst of the instant invention.

## EXAMPLE I

To a suspension of 182 g of $V_2O_5$ in 800 ml of water is added 378 g of oxalic acid, $H_2C_2O_4 \cdot 2H_2O$, dissolved in 800 ml of water in small portions with vigorous stirring. After all the $V_2O_5$ has dissolved, the deep blue vanadyl oxalate solution is filtered, and the water removed from the recovered filtrate on a rotary evaporator under reduced pressure. The recovered blue solid vanadyl oxalate is placed in an inert atmosphere furnace and the furnace purged with nitrogen for several minutes. The temperature is then raised to 400°C. over a period of about 4 hours, kept at 400°C. for about 16 hours, and then cooled to room temperature still under a nitrogen atmosphere. The recovered powder is mixed with 2 weight % graphite and pressed into 3.12 mm diameter x 3.12 mm long pellets. The procedure is repeated until sufficient catalyst is produced to make a catalyst charge.

The following three examples illustrate other methods for preparing reduced vanadium oxide catalysts for the oxidation of lower aliphatic hydrocarbons.

## EXAMPLE II

To 182 g of $V_2O_5$ slurried in 1 liter of 90% formic acid at about 50°C. is added dropwise with rapid stirring over a period of two hours, 60 ml of 64% hydrazine in water. After addition is complete, the reaction mixture is stirred for an additional two hours at about 80°C. The resulting

green, fine crystalline precipitate is collected by filtration, washed several times with formic acid, acetone, and air dried. The recovered vanadyl formate is placed in an inert atmosphere furnace and the furnace purged with nitrogen for several minutes. The temperature is then raised to 400°C. over a period of about 4 hours, kept at 400°C. for about 16 hours, and then rapidly cooled to room temperature still under a nitrogen atmosphere. The recovered powder is mixed with 2 weight % graphite and pressed into 3.12 mm diameter x 3.12 mm long pellets. The procedure is repeated until sufficient catalyst is produced to make a catalyst charge.

EXAMPLE III

About 900 g of $V_2O_5$ are mixed with 2 weight % graphite and pressed into 3.12 mm diameter x 3.12 mm long pellets. The pellets are placed in a controlled atmosphere furnace and the furnace purged with hydrogen. Under a steady flow of hydrogen the temperature is raised to 500°C., held at the temperature for about 5 hours, and then cooled to room temperature still under a steady stream of hydrogen. The extent of reduction can be measured by analyzing for water in the vent gas stream.

EXAMPLE IV

To 200 g of vanadyl sulfate dissolved in 1 liter of water is added with stirring ammonium hydroxide with ice bath cooling until a pH of about 5-6 is reached. The resulting brown precipitate of vanadyl hydroxide is collected by filtration, washed several times with water, and dried at 110°C. in a forced draft oven. The dried powder is mixed with 2 weight % graphite and pressed into 3.12 mm diameter x 3.12 mm long pellets. The pellets are

0002681

placed in an inert atmosphere furnace and the furnace purged with nitrogen. The temperature is then raised to 400°C. over a period of about 4 hours, held at that temperature for about 16 hours, and then cooled to room temperature still under a nitrogen atmosphere. The procedure is repeated until sufficient catalyst is produced to make a catalyst charge.

EXAMPLES V-VIII

The following examples comparatively illustrate the superior results utilized by the preferred catalyst of the instant invention in the high pressure, high temperature vapor phase oxidation of n-butane to acetic acid as compared to the reduced vanadium catalyst prepared by prior art methods.

A 19.1 mm schedule 40 steel pipe is employed to hold the catalyst charge. The pipe is about 1.5 m long with an inside diameter of about 21 mm. The usual catalyst charge is about 250 $cm^3$ of pelleted catalyst. The catalyst is usually diluted heterogeneously with an equal volume of about the same size pellets of silicon carbide. The reactor is heated to the desired temperature using pressurized steam. Flow rates of the lower aliphatic hydrocarbon and air (or oxygen) are determined by mass flowmeters. Steam is introduced as water at a known flow rate and flashed to steam with the flow rate of steam calculated by application of the ideal gas law. After the flow rates are stabilized, the temperature of the reactor (initially about 240°C.) is then slowly raised until the desired oxygen conversion rate is achieved. Material balances are then obtained at this temperature. Reactions are conducted under pressure so that cooling water can

be used to condense the butane recycle stream. Analysis of the vent gas stream entails passing the reaction products plus unreacted lower aliphatic hydrocarbon and steam through a water-cooled condenser after leaving the heated reaction zone to remove the liquid products and water from the vent stream. The vent stream leaving the condenser, now containing primarily lower aliphatic hydrocarbons, carbon oxides, and nitrogen (if air is used) is analyzed by standard gas chromatographic techniques. Components analyzed for include butane, butenes, acetaldehyde, oxygen, nitrogen, carbon monoxide, and carbon dioxide. The liquid product is collected at the end of the run and analyzed by standard gas chromatographic techniques. Components analyzed for include: acetic acid, acetaldehyde, propionic acid, maleic acid, formaldehyde, formic acid, and acetone.

The following represents typical results realized when the catalysts of examples 1-4 are utilized in the above-described process.

| Catalyst Example | 5 | 6 | 7 | 8 |
|---|---|---|---|---|
| Prepared via | Vanadyl Oxalate | Vanadyl Formate | $H_2$ Reduced $V_2O_5$ | Vanadyl Hydroxide |
| Reactor Pressure, bar | 9.65 | 9.79 | 9.65 | 9.65 |
| Reactor Temperatur, $^{o}C$ | 264 | 250 | 272 | 269 |
| Feed Stream (Mol Ratio) $C_4^o:O_2:H_2O$ | 9.1:1.0:8.0 | 9.0:1.0:7.9 | 9.2:1.0:8.1 | 9.4:1.0:7.9 |
| Total Feed Rate 1/min. (STP) | 28.99 | 28.57 | 29.12 | 29.29 |
| Butane Conversion, % | 3.5 | 4.0 | 3.5 | 3.5 |
| Oxygen Conversion, % | 98 | 99 | 93 | 98 |
| Carbon Efficiency, % | | | | |
| Butene | 11 | 27 | 26 | 29 |
| Acetic Acid | 51 | 34 | 38 | 36 |
| Acetaldehyde | 5.2 | 7.8 | 7.7 | 8.0 |
| Carbon Oxides | 29 | 27 | 22 | 22 |

 

EXAMPLE IX

The following illustrates preparation of the preferred catalyst of the instant invention supported on a porous silica carrier.

Silica spheres (215 g, about 600 $cm^3$ of 3.18 mm to 6.35 mm diameter spheres) are impregnated using standard techniques with a 4 molar aqueous solution of vanadyl oxalate. The water is removed on a rotary evaporator under reduced pressure. The spheres are placed in a 5 cm diameter Pyrex tube, the tube purged with nitrogen for several minutes, and then heated to $200^oC$. The temperature is then increased to $400^oC$. over a period of about 5 hours, held at that temperature for about 16 hours, and then cooled to room temperature still under a nitrogen atmosphere. This procedure is repeated twice to achieve a loading of about 25 weight percent vanadium on the silica spheres.

EXAMPLE X

The following represents typical results when the catalyst of Example IX is utilized in the process of Examples V through VIII. About 420 $cm^3$ of supported catalyst is used without any silicon carbide diluent.

| | |
|---|---|
| Reactor Pressure, bar | 9.65 |
| Reactor Temperature ($^oC$) | 266 |
| Feed Stream (Mole Ratio) | |
| $C_4^o : O_2 : H_2O$ | 7.0 : 1.0 : 6.2 |
| Total Feed Rate | |
| 1/min (STP) | 29.81 |
| Butane Conversion (%) | 4.6 |
| Oxygen Conversion (%) | 99 |

| Carbon Efficiency (%) | |
|---|---|
| Butenes | 18 |
| Acetic Acid | 42 |
| Acetaldehyde | 5.2 |
| Carbon Oxides | 29 |

EXAMPLE XI

The following illustrates the preferred catalyst of the instant invention bound in a silica matrix for use in a fluid bed operation.

Vanadium pentoxide (364 g) is thoroughly mixed with 794 g of oxalic acid ($H_2C_2O_4 \cdot H_2O$) as dry powders and placed in a large beaker. The beaker is immersed in an oil bath heated to 160°C. The reaction begins almost immediately as evidenced by gas evolution. After the reaction is completed the reaction mixture is held at 160°C. for an additional one hour. The resulting blue solid vanadyl oxalate is removed, placed in a 5 cm diameter quartz tube, and the tube purged with nitrogen and then heated to 200°C. The temperature is then increased to 425°C. over a period of about 5 hours, held at that temperature for about 14 hours, and then cooled to room temperature still under a nitrogen atmosphere. This procedure is repeated until about 1360 g of reduced vanadium oxide is recovered. The reduced vanadium oxide (1360 g) is passed through an U. S. Standard sieve with sieve openings of 150 µm and combined with 1283 g of DuPont's Ludox AS-30, an ammonia stabilized silica sol, in a blender to form a thick uniform paste. The water is removed on a rotary evaporator under reduced pressure. The recovered black solid is then calcined under nitrogen at 415°C. for about 16 hours. The resulting silica bound reduced vanadium oxide catalyst is passed through an U. S. Standard sieve with sieve openings of 250 µm and that portion retained on an

U. S. Standard sieve with sieve openings of 38 μm is used as the catalyst charge.

EXAMPLE XII

The following represents typical results when the catalyst of Example XI is utilized in the process of Example V with the following changes: The reactor consists of a 3 m long x 31.75 mm schedule 40 steel pipe with a 35 mm inside diameter and equipped with a 61 cm x 15.24 cm inside diameter extension for catalyst disengagement. The gaseous feed stream is preheated and then fed into the bottom of the fluid bed reactor through a porous metal disc. About 670 $cm^3$ of fluid catalyst is used.

| | |
|---|---|
| Reactor Pressure, bar | 11 |
| Reactor Temperature, $^oC$ | 269 |
| Feed Stream (Mol Ratio) | |
| $C_4^o : O_2 : H_2O$ | 7.6 : 1.0 : 4.1 |
| Total Feed Rate | |
| 1/min (STP) | 42.83 |
| Butane Conversion (%) | 4.4 |
| Oxygen Conversion (%) | 98 |
| Carbon Efficiency (%) | |
| Butenes | 20 |
| Acetic Acid | 49 |
| Acetaldehyde | 4.0 |
| Carbon Oxides | 24 |

EXAMPLE XIII

The following illustrates another catalyst of the instant invention - vanadyl tartrate - bound in a silica matrix for use in a fluidized bed operation.

Vanadium pentoxide (364 g) is slurried in 800 ml. of water and to it is added 450.2 g of

tartaric acid dissolved in 1400 ml. of water. About one-half the tartaric acid solution is added initially, the reaction mixture heated to about 80°C., and the rest of the tartaric acid solution is then added in small portions. After addition is complete, the now deep bluish-purple vanadyl tartrate solution is held at about 80°C. for 2 hours, filtered, and the recovered filtrate reduced in volume to 1000 ml.

Using standard catalyst impregnation techniques, the vanadyl tartrate solution (380 ml) is combined with 800 $cm^3$ (286 g) of a silica fluid bed support. After drying at 50°C. for about 16 hours, the impregnation procedure is repeated using 150 ml. of vanadyl tartrate solution. The impregnated supported catalyst is dried again at 50°C. for about 16 hours and then calcined at 400°C. under a nitrogen atmosphere for about 16 hours.

EXAMPLE XIV

The following represents typical results when the catalyst of Example XIII is utilized in the process of Example XII.

| | |
|---|---|
| Reactor Pressure, bar | 6.9 |
| Reactor Temperature, °C | 258 |
| Feed Stream (Mol Ratio) | |
| $C_4^o : O_2 : H_2O$ | 8.2 : 1.0 : 4.0 |
| Total Feed Rate | |
| 1/min (STP) | 14.18 |
| Butane Conversion (%) | 3.2 |
| Oxygen Conversion (%) | 99 |
| Carbon Efficiency (%) | |
| Butenes | 13 |
| Acetic Acid | 50 |
| Acetaldehyde | 2.7 |
| Carbon Oxides | 32 |

EXAMPLES XV - XVI

The following two examples run with neat $V_2O_4$ catalyst illustrate the criticality of reactor temperature when operating the high pressure reaction of the instant invention. A 140 cm reactor similar to that in Examples V through VIII is used.

| | | |
|---|---|---|
| Reactor Temperature ($^oC$) | 225 | 240 |
| Reactor Pressure, bar | 9.65 | 9.65 |
| Peak Temperature Location (cm into catalyst bed) | 102 | 43.2 |
| Catalyst Oxidation (Visible) | Severe | Some |
| Reactant Feed Rate (liters/min) (STP) | | |
| Butane | 14.48 | 14.48 |
| Oxygen | 2.12 | 2.14 |
| Steam | 14.62 | 14.35 |
| Total Feed Rate | 31.22 | 30.97 |
| Butane Conversion (%) | 5.4 | 5.7 |
| Oxygen Conversion (%) | 99.0 | 99.6 |
| Carbon Efficiency (%) | | |
| Butene | 33.41 | 38.27 |
| Acetic Acid | 30.32 | 26.08 |
| Acetaldehyde | 9.25 | 9.85 |
| Carbon Oxides | 23.69 | 22.28 |

When the reactor is operated at about 240$^oC$., the peak temperature occurs about 43.2 cm from the inlet of the reactor. When the reactor is operated at about 225$^oC$., the peak temperature occurs about 102 cm into the catalyst bed and the oxidation of the catalyst in the beginning of the bed is quite visible when said catalyst is removed from the reactor because when the catalyst is in the reduced state it is dark blue-black. When it is in the oxidized state, it is yellow.

THE CLAIMS:

1. Vanadium oxide catalyst characterized in that the vanadium oxide is a reduced oxide in which the vanadium ions have a valence less than five, the average valence of the vanadium ions generally ranges from 3 to about 4.5.

2. The catalyst of Claim 1 characterized in that said catalyst has been prepared by the thermal decomposition of a water soluble salt of vanadium and a multibasic organic acid.

3. A process for preparing the catalyst of Claim 1 and 2 characterized by the steps of:

solubilizing vanadium pentoxide with a multibasic organic acid;

drying; and

thermally decomposing same.

4. A process for preparing the catalyst of Claim 1 and 2 characterized by the steps of:

intimately reacting at high temperatures vanadium pentoxide with a multibasic organic acid powder; and

thermally decomposing same.

5. A process for preparing the catalyst of Claim 1 and 2 characterized by the steps of:

intimately reacting at high temperature vanadium pentoxide and a multibasic organic powder at high temperatures;

decomposing same;

intimately blending with an aqueous ammonia stabilized silica sol;

drying; and

calcining the dried product.

6. The use of the catalyst according Claim 1 to 5 for preparing acetic acid by the high pressure vapor phase oxidation of lower aliphatic hydrocarbons characterized in that a catalytic amount of said catalyst is present in the lower aliphatic hydrocarbon feed stream comprising alkanes with an oxygen-containing gas in the vapor phase in the presence of steam.

7. The use of the catalyst according Claim 1 to 5 in the acetic acid reaction of Claim 6 wherein the temperature of the process is from about 220 to about 400°C., typically from greater than 240 to about 350°C., preferably from greater than 250 to about 300°C.

8. The use of the catalyst according Claim 1 to 5 in the acetic acid reaction of Claim 6 wherein the pressure of the process is from greater than 1 to about 50 bars, typically from greater than 1 to about 30 bars, preferably from about 5 to about 20 bars.

9. The use of the catalyst according Claim 1 to 5 in the acetic acid reaction of Claim 6 wherein the molar ratio of oxygen to lower aliphatic hydrocarbon is from about 0.05 to 0.30.

10. The use of the catalyst according Claim 1 to 5 in the acetic acid reaction of Claim 6 wherein the molar ratio of steam to lower aliphatic hydrocarbon is from about 0.1 to 2.0, preferably from about 0.5 to 1.2.